# EUROPEAN PATENT SPECIFICATION

(11) **EP 0 369 687 B1**
(45) Date of publication and mention of the grant of the patent: **03.04.1996**
(21) Application number: 89311637.6
(22) Date of filing: 10.11.1989
(51) Int. Cl.: C07D 463/00

(54) **Solvates of a beta-lactam antibiotic**
Solvate von einem Beta-Lactam-Antibiotikum
Solvates d'un antibiotique de bêta-lactame

(30) Priority: 14.11.1988 US 271545
(43) Date of publication of application: 23.05.1990
(73) Proprietor: ELI LILLY AND COMPANY, Indianapolis, Indiana 46285 (US)
(72) Inventor: Eckrich, Thomas Michael, IndianapolisIndiana 46217 (US); Hoying, Richard Charles, IndianapolisIndiana 46227 (US)
(74) Representative: Tapping, Kenneth George

(56) References cited:
- EP-A- 0 014 476
- EP-A- 0 311 366
- EP-A- 0 327 364

## Description

This invention relates to novel solvate forms of a β-lactam antibiotic, more particularly to novel crystalline bis(DMF), dihydrate mono(DMF) and mono(DMF) forms of the p-lactam antibiotic of the Formula I 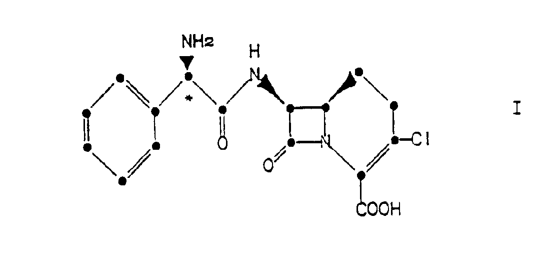 is a potent orally-active agent. The antibiotic isdescribed, for example, byJ. Hashimoto et al. in U.S. Patent No. 4,335,211, issued June 15, 1982. For brevity's sake, the compound of Formula I will be referred to by the serial number LY163892.

The present invention is directed to the crystalline bis(N, N'-dimethylformamide) solvate of LY163892, and the crystalline mono(N,N'-dimethylformamide) solvate of LY163892. The two solvates will be referred to hereinafter as the "bis(DMF)", and the "mono(DMF)" solvates, respectively It will be understood that these abbreviated terms refer to the crystalline or microcrystalline form of the three solvates.

The bis(DMF) and mono(DMF) solvates are convenient intermediates to LY163892 in general and to the monohydrate form of LY163892 specifically LY163892 monohydrate ("monohydrate") is a pharmaceutically elegant hydrate of LY163892. The monohydrate affords a stable, easy-to-handle form of LY163892, a compound that heretofore was both difficult to purify and obtain in a pharmaceutically elegant form. The monohydrate also affords a necessary form of LY163892 useful in the manufacture of the various dosage forms of the antibiotic. The monohydrate is disclosed in EP-A-0311366.

One aspect of the present invention is directed to the crystalline bis(DMF) solvate of LY163892. The formula for LY163892 is given below as Formula I. More specifically, the invention is directed to crystalline LY163892 bis(DMF) having the X-ray powder diffraction pattern listed below in Table 1.

Yet another aspect of the invention is the crystalline mono(DMF) solvate of LY163892. A preferred form of the mono(DMF) solvate is a crystalline compound have the X-ray powder diffraction pattern set forth in Table 3 below.

The instant invention is directed to the crystalline bis(DMF), dihydrate mono(DMF) and mono(DMF) solvates of the compound of Formula I:

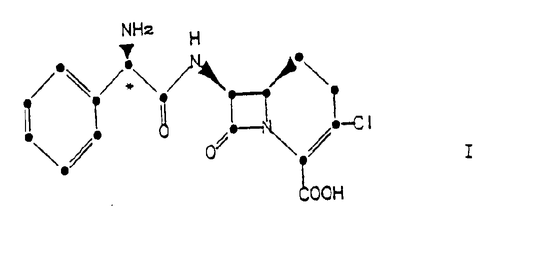

In the present solvates of Formula I the C-2' asymmetric center has the R absolute configuration. Furthermore, the instant solvates may encompass the zwitterionic form of the compound of Formula I.

A first embodiment of the invention is a crystalline bis(DMF) solvate of LY163892 exhibiting the X-ray powder diffraction pattern of Table 1:

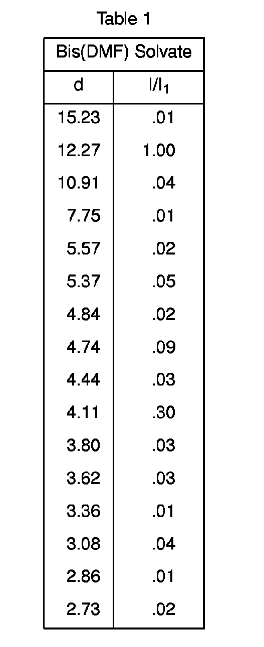

The diffraction pattern in Table 1 was obtained with nickel-filtered copper radiation (Cu:Ni) of wavelength λ = 1.5406 Å. The interplanar spacings are in the column marked "d" and are in Angstroms and the relative intensities are in the column marked "I/I₁".

A second embodiment of the invention is the crystalline mono(DMF) solvate of LY163892 exhibiting the X-ray powder diffraction pattern set forth below in Table 3:

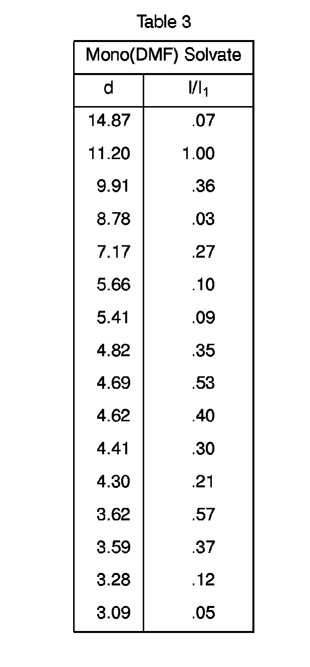

The X-ray data in Table 3 was collected employing the same instrument parameters used to collect the data in Table 1 except that λ = 1.5418 A.

The bis(DMF) solvate of LY163892 can be readily prepared by suspending any form of LY163892, for example the anhydrate, ethanol solvate, in aqueous DMF and forming a solution. A solution is most commonly effected by the addition of acid, typically dilute hydrochloric acid, although solution may also be caused by addition of base. The desired bis(DMF) solvate is precipitated by the adjustment of the pH of the solution to approximately 5 to 6 by the addition of acid or base as needed, at a temperature in the range of about 45°C to about 55°C, most preferably at about 50°C. The precipitated solid is collected, typically by filtration, and vacuum dried to provide the bis(DMF) solvate of the invention.

The two solvates of the present invention may also be prepared by acylating a 7p-amino ("nucleus") compound of the Formula II 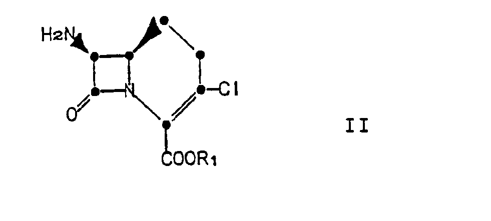 where R₁ is a carboxy-protecting group with an acylating agent of the Formula III 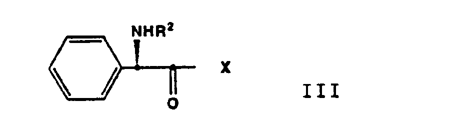 where X is a leaving group and R₂ is hydrogen or an amino protecting group, in DMF followed by deprotection. The nucleus and its synthesis are disclosed in U.S. Patent No. 4,734,494, herein incorporated by reference.

The carboxy-protecting group R₁ of Formula II is a conventional carboxy-protecting group and preferably one which is not sterically hindered. Examples of such groups are alkyl, benzyl and substituted benzyl groups such as 4-methoxybenzyl, 4-nitrobenzyl, 4-methylbenzyl, 3,5-dimethylbenzyl, and 4-chlorobenzyl; silyl group such as a trialkylsilyl group (trimethylsilyl); and halo-substituted alkyl groups such as the 2,2,2-trichloroethyl, 2,2,2-tribromoethyl, and 2-iodoethyl groups. A preferred ester group is the benzyl or a substituted benzyl ester group. The amino protecting group R₂ of Formula II is selected from either the carbamates such as t-butoxycarbonyl or benzyloxycarbonyl, or the enamines.

In particular, the acylation of the p-nitrobenzyl nucleus compound (Rᵢ = para-nitrobenzyl) takes place in cooled (for example, -20°C) DMF. The acylating agent, an activated derivative of 2-(R)-2-phenyl-2-aminoacetic acid, is added to the cooled DMF. A preferred acylating agent is a compound of the Formula IV

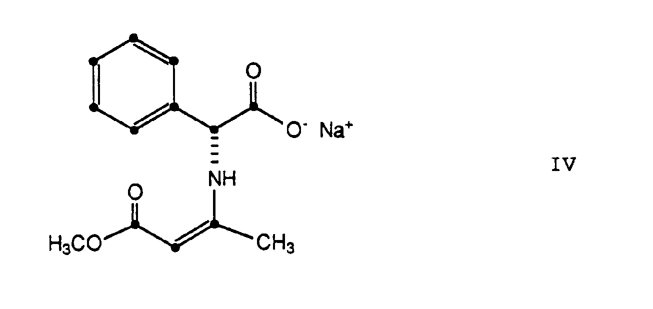

This compound may be prepared according to the procedure of Dane et al. in Angew. Chem., Vol. 74, 873 (1962).

The reaction solution is cooled, and when IV is the acylating agent, methanesulfonic acid, dimethylbenzylamine, and methyl chloroformate are added in rapid succession. The solution is stirred and maintained at a very low (approximately -45°C) temperature, then the pNB ester of the nucleus is added with stirring. The reaction is stirred at low temperature (for example, -45°C) until the acylation reaction is substantially complete (as determined by conventional means such as thin layer chromatography). The mixture is then warmed slowly to approximately 0°C and the reagents for removing the amino and carboxy protecting groups (such as water, concentrated hydrochloric acid, and zinc dust for the pNB ester) are added slowly while maintaining the initial temperature of the solution. The solution is stirred at room temperature until the reaction is complete. In the case where R₁ was the pNB ester the pH is raised (for example to 2.3) by the addition of a base such as triethylamine, and the resultant zinc residue is removed by filtration. The pH is gradually taken higher until a white suspension is formed and the pH remains stable without the addition of base (typically around pH 5.6). (The mixture may be seeded with LY163892 at about pH 4.6 to induce crystallization). The solid phase of the suspension is collected by filtration. The wet filter cake is suspended in a 90:10 mixture of 9:1 DMF/H₂O and solution effected with concentrated hydrochloric acid. The solution is cooled and the pH raised in small increments with a base (triethylamine) until a suspension forms and the pH of the solution is stabilized with further additions of base (for example, to a pH of approximately 5.6). The crystals are again collected by filtration and dried to give the bis(DMF) solvate.

Alternatively, the bis(DMF) solvate can be made from a concentrated DMF solution of mono(DMF) solvate. Specifically, anti-solvent (preferably acetonitrile) is added in equal volume to the concentrated DMF solution and the mixture is cooled (for example, to 0°C). The solid bis(DMF) precipitate is collected by filtration as above.

The methods for the acylation of the 7p-amino compounds of Formula II with an acyl side chain are similar to the methods for the acylation of 6-aminopenicillanic acid, 7-aminodesacetoxycephalosporanic acid, and 7-aminocephalosporanic acid. One method is to simply combine the 7p-amino nucleus with an acid chloride or acid bromide in the presence of an acid scavenger. The acid chloride or acid bromide may be formed in situ. Another method is to combine the 7p-amino nucleus with the free carboxylic acid form of the side chain (or its acid salt) and a condensing agent. Suitable condensing agents include N,N'-disubstituted carbodiimides such as N,N'-dicyclohexylcarbodiimide, N,N'- diethylcarbodiimide, N,N'-di(n-propyl)-carbodiimide, N,N'-di(isopropyl)carbodiimide, N,N'-diallylcarbodiimide, N,N'- bis(p-dimethylaminophenyl)carbodiimide, N-ethyl-N'-(4_{"}-ethylmorpholinyl)carbodiimide, and the like. Other suitable carbodiimide condensing agents are disclosed by Sheehan in U.S. Patent No. 2,938,892 and by Hofmann et al. in U.S. Patent No. 3,065,224. Azolides, such as N,N'-carbonyldiimidazole and N,N'-thionyldiimidazole, may also be used as condensing agents. Dehydrating agents such as phosphorus oxychloride, the alkoxyacetylenes, and 2-halogenopyrid- inium salts (such as 2-chloropyridinium methyl iodide, 2-fluoropyridinium methyl iodide, and the like) may be used to couple the free acid or its acid salt with the 7p-amino nucleus.

Another acylation method entails first converting the free carboxylic acid form (or the corresponding salt) of the acyl side chain to the corresponding active ester derivative, which is in turn used to acylate the nucleus. The active ester derivative is formed by esterifying the free acid form with groups such as p-nitrophenol, 2,4-dinitrophenol, trichlorophenol, pentachlorophenol, 2-chloro-4,6-dimethoxytriazene, N-chlorosuccinimide, N-chloro maleic imide, N-chlorophthalimide, 1-hydroxy-1H-benzotriazole or 1-hydroxy-6-chloro-1H-benzotriazole. The active ester derivatives can also be mixed anhydrides, which are formed with groups such as methoxycarbonyl, ethoxycarbonyl, isobutoxycarbonyl, trichloromethylcarbonyl, and isobut-2-ylcarbonyl, and the carboxylic acid of the acyl side chain. The mixed anhydrides are synthesized by acylating the carboxylic acid of the acyl side chain.

Alternatively, the 7p-amino nucleus can be acylated with the N-ethoxycarbonyl-2-ethoxy-1,2-dihydroquinoline (EEDQ) derivative of the acyl side chain. In general, the free acid form of the acyl side chain and EEDQ are reacted in an inert, polar organic solvent (such as tetrahydrofuran, acetonitrile, and the like). The resultant EEDQ derivative is used in situ to acylate the 7p-amino nucleus.

Yet another method of acylating the 7p-amino compounds entails the use of an enzymatically-assisted process. Such a process is described in Hashimoto et al., U.S. Patent No. 4,335,211, issued June 15, 1982, herein incorporated by reference.

A preferred method of acylation in general is to first silylate the nucleus with, for example, N,N'-bis(trimethylsilyl)urea (BSU) in DMF. The DMF solution is cooled to a low temperature (-45°C to -50°C) then pyridine and the hydrochloride salt of the acid chloride derivative of phenylglycine are added. The acylation is quenched by the addition of strong (5 or 6N) hydrochloric acid, and filtered. The acylated product is then recovered by adjusting the pH of the reaction solution to approximately 6 (more preferably 6.1) by the addition of a base such as triethylamine. The solution is usually seeded with a small amount of LY163892 (such as the mono DMF dihydrate form) after the initial addition of triethylamine. The crystals are collected by filtration. Further details on these and other procedures for the acylation are given below in the Experimental Section.

The amino- and carboxy-protecting groups are removed by methods well known in the art. Examples of conditions for the removal of these two types of protecting groups can be found in standard works on the subject, such as E. Haslam, "Protective Groups in Organic Chemistry", J.G.W. McOmie, Ed., Plenum Press, New York, N.Y, 1973, Chapters 2 and 5, and T.W. Greene, "Protective Groups in Organic Synthesis", John Wiley and Sons, New York, N.Y, 1981, Chapters 5 and 7, respectively.

As noted above, LY163892 bis(DMF), and mono(DMF) solvates are useful as intermediates to LY163892 monohydrate. The monohydrate is prepared by first suspending any of the above starting materials in water. The most common procedure is to effect solution of the starting material by the addition of a minimum amount of acid, generally 6N (or more dilute) hydrochloric acid. Alternatively, a solution of the starting material is effected by adding the minimum amount of base (for example, the minimum amount of 2N sodium hydroxide, resulting in a pH of about 7.6).

Regardless of how solution is effected, crystallization is induced by slowly adjusting the pH of the solution of starting material to approximately 4, and preferably 4.8. For example, if the solution is effected by the addition of acid, it is preferred to raise the temperature of the solution to about 50°C and slowly add triethylamine (alternatively sodium hydroxide) to the solution until a pH of approximately 4.8 is obtained. The gradually developing suspension is stirred and maintained at about 50°C during the addition of the base. Seeding the solution with a small amount of crystalline monohydrate early in the addition period of base is preferred. For example, seeding is often done when the pH of the solution is approximately 1.8. If the starting material solution was effected by the addition of base, the pH is slowly taken to approximately 4 by the addition of an acid (preferably 2N hydrochloric acid). Effecting solution of the starting material with hydrochloric acid and inducing crystallization by adjusting the pH of the solution to approximately 4.8 with triethylamine is preferred.

The suspension resulting from adjusting the pH of starting material solution to approximately 4 is isolated by conventional filtering techniques, such as vacuum filtration on a Buchner funnel. The collected crystals are washed and allowed to dry in air at ambient temperature. Alternatively, the warm pH-adjusted suspensions (50°C) are cooled to approximately 20°C, stirred, filtered (such as on Buchner funnel) and the collected solid dried at 30°C for 24 to 48 hours by conventional means (such as a cleaned-air oven).

As further aspects of the present invention there is provided a process for preparing a crystalline bis (N,N'-dimethylformamide) solvate form of the compound of the Formula I which comprises dissolving a compound of Formula I in aqueous N,N'-dimethylformamide, adjusting the pH to approximately 5 to 6, followed by filtration and vacuum drying.

As a further aspect of the present invention, these is provided a process for preparing a crystalline mono(N,N'- dimethylformamide) solvate form of the compound of Formula I which comprises dissolving a compound of Formula I in aqueous N,N'-dimethylformamide, adjusting the pH to about 5.9, adding acetonitrile, followed byfiltration of the resulting solid and air drying at slightly above room temperature.

In the following Examples, the terms dimethylformamide, nuclear magnetic resonance spectra, mass spectrum and infrared spectroscopy are abbreviated DMF, NMR, MS and IR, respectively

In conjunction with the NMR spectra, the following abbreviations are used: "s" is singlet, "d" is doublet, "t" is triplet, "q" is quartet, and "m" is multiplet.

The NMR spectra were obtained on a General Electric QE-300 300 MHz instrument. The chemical shifts are expressed in ppm values (parts per million downfield from tetramethylsilane).

### Experimental Section

### Example 1

### Synthesis of LY163892 Mono(DMF)

To a solution of 30 g (138.5 mmol) of 7f3-amino-3-chloro-3-(1-carba-1-dethiacephem)-4-carboxylic acid in 500 ml of DMF containing 0.03% water was added approximately 10 drops of trimethylsilyl chloride followed by 32.85 g of N,N'- bis(trimethylsilyl)urea. The reaction mixture was stirred for 90 minutes at room temperature and cooled to approximately -50°C. To the mixture was added 10.8 ml (10.6 g, 122.7 mmol) of pyridine followed by 27.07 g (131.5 mmol) of 2-(R)-2-phenyl-2-aminoacetyl chloride hydrochloride. The mixture was stirred at about -35°C for approximately one hour and cooled to -50°C. To the mixture was added 7.32 ml of methanol in 30 ml of DMF. The reaction mixture was stirred to 0°C over a period of 40 minutes and 54 ml of water was added. The reaction mixture was allowed to warm to about 15°C over the next 30 minutes and triethylamine was slowly added until the pH of the mixture was about 3.2. The filtrate was warmed to about 50°C. To the mixture was added triethylamine until the pH reached 4.6 and the mixture was allowed to stand at room temperature for one hour. The mixture was then stirred at about 40°C and a mixture of DMF: triethylamine (1:1,v:v) was added until the pH was about 5.9. The mixture was cooled to 25°C and stirred for 20 minutes. To the mixture was added 500 ml of acetonitrile and the resulting mixture was stirred for 30 minutes. The mixture was filtered and the precipitated solid was dried in an air oven at 30°C until the weight of the solid was constant to provide LY163892 mono(DMF).
H-NMR (300 MHz, D₂0/DC1): 8.18 ppm (s, 1 H); 7.79 (s,1 H); 5.65 (d, J=4.8 Hz, 1 H); 5.51 (s, 1 H); 4.21 (d of t, 1 H); 3.28 (s, 3H); 3.12 (s, 3H); 2.84 (m, 2H); 1.95 (m, 1H); 1.58 (m, 1 H).
¹3C-NMR (75.48 MHz, D₂O/DCl): 21.99, 31.85, 32.27, 37.77, 53.47, 57.37, 58.57, 123.5, 128.9, 130.6, 131.4, 132.5, 133.8, 164.4, 165.7, 166.3, 169.9 ppm.
I R (KBr disc): 2950-3620 cm-¹, (m and broad) 1772.7, 1691.7, 1658.9, 1598, 1566, 1409, 1389, 1378, 1349, 1325 (all medium to strong).
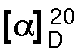 = +17.85°, c = 1.02 in 0.1 N HCI.
MS =350, 352

### Example 2

### Synthesis of LY163892 bis(DMF)

Powdered sodium 2-(R)-2-phenyl-2-(((Z)-methyl but-2-en-3-yloate)amino)acetate (4.59 g, 16.92 mmol) was sifted into a 250 ml round bottom flask containing 75 ml of stirred DMF under a nitrogen atmosphere. Next 22 µl of methanesulfonic acid was added to the reaction mixture which was cooled to about -45°C. To the mixture was added 47.6 µl of dimethylbenzylamine followed by 1.53 g (1.25 ml, 16.15 mmol) of methyl chloroformate. The reaction mixture was stirred at about -45°C for approximately 50 minutes. To the mixture was added a solution of 25 ml of DMF and 5.41 g (15.38 mmol) of 7β-amino-3-chloro-3-(1-carba-1-dethiacephem)-4-carboxylic acid (4-nitrophenyl)methyl ester dropwise to the reaction mixture. The mixture was stirred for two hours and warmed to about 0°C for approximately 45 minutes. While maintaining the temperature of the reaction mixture between about 5°C and 10°C, 6.89 ml of water, 12.32 ml of concentrated hydrochloric acid, 3.55 g of zinc dust and 8.93 ml of concentrated hydrochloric acid were added to the reaction mixture. The mixture was stirred at room temperature for about five hours and the pH was adjusted to 2.35 with triethylamine. The mixture was filtered and the pH was again adjusted to 4.6 with triethylamine. The mixture was stirred for 45 minutes and the pH was raised to 5.75 with triethylamine. The mixture was stirred for an additional 15 minutes. The precipitated solid was collected by vacuum filtration and washed with 30 ml of DMF:water (9:1, v:v). The solid was vacuum dried for approximately 10 hours to provide 4.8 g of the bis(DMF) solvate of LY163892.

This material was further purified by suspending the material in 40 ml of DMF:water (9:1, v:v). The solution was cooled to 10°C and the pH was adjusted to 1.7 with concentrated hydrochloric acid. The mixture was filtered and the pH was raised to 5.6 with triethylamine. The precipitated solid was collected by vacuum filtration, washed with DMF:water (9:1, v:v) and vacuum dried at 26°C to provide 3.72 g of bis(DMF) LY163892.
H-NMR (300 MHz, D₂0/DCI): 8.18 ppm (s, 2H); 7.79 (s,1 H); 5.65 (d, 1 H); 5.51 (s, 1 H); 4.21 (d of t, 1 H); 3.28 (s, 6H); 3.12 (s, 6H); 2.84 (m, 2H); 1.95 (m, 1 H); 1.58 (m, 1 H).
IR (KBr disc): 1772.7 cm⁻¹, 1691.7, 1659.9, 1599.1, 1566.3, 1407.2, 1389.8, 1378.2, 1349.3, 1325.2 (all medium to strong)
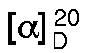 = +12.68°, c = 0.35 in 0.01 N HCI.
MS = 350, 352

### Example 3

### Conversion of LY163892 bis(DMF) Solvate to LY163892 Monohydrate

The pH of a suspension of 4.0 g of bis(DMF) LY163892 in water was adjusted to 1.57 with concentrated hydrochloric acid. This mixture was seeded with LY163892 monohydrate. The pH was adjusted to approximately 4.9 while maintaining the temperature of the mixture at approximately 50°C. The mixture was cooled to room temperature and the precipitated solid was collected by vacuum filtration, washed with 6 ml of water and air dried. An X-ray powder diffraction pattern of the isolated product was identical to an authentic reference standard. Karl Fischer analysis: 4.36% moisture

## Claims

1. A crystalline bis (N,N'-dimethylformamide) solvate form of the compound of the Formula I 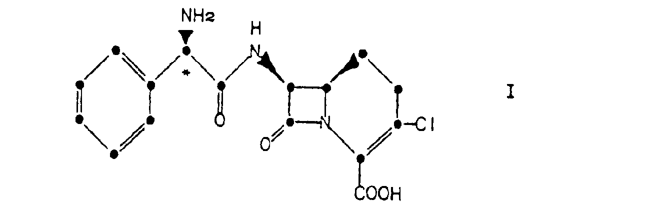 which has the following X-ray powder diffraction pattern obtained with a nickel-filtered copper radiation of λ = 1.5406 A wherein d represents the interplanar spacing and I/I₁ the relative intensity:

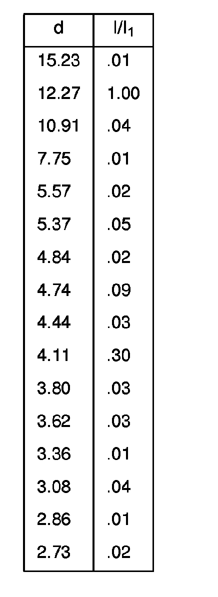

2. A crystalline mono(N,N'-dimethylformamide) solvate form of the compound of Formula I: 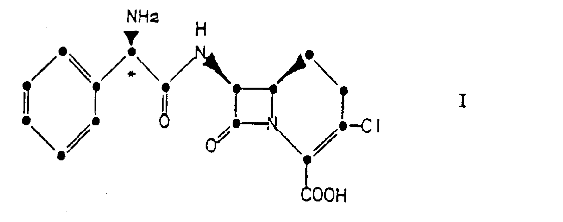 which has the following X-ray powder diffraction pattern obtained with a nickel-filtered copper radiation of λ = 1.5418 A wherein d represents the interplanar spacing and I/I₁ the relative intensity:

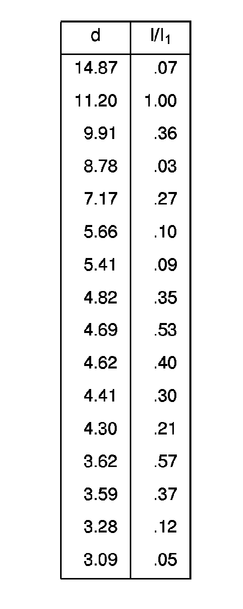

3. A process for preparing a crystalline bis (N,N'-dimethylformamide) solvate form of the compound of the Formula I 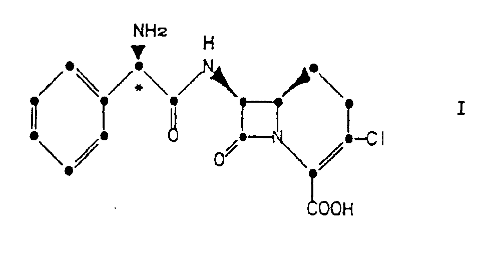 which comprises dissolving a compound of Formula I in aqueous N,N'-dimethylformamide, adjusting the pH to 5 to 6, followed by filtration and vacuum drying.

4. A process according to Claim 3 for preparing a compound of Claim 1, which has the following X-ray powder diffraction pattern obtained with a nickel-filtered copper radiation of λ = 1.5406 A wherein d represents the interplanar spacing and I/I₁ the relative intensity:

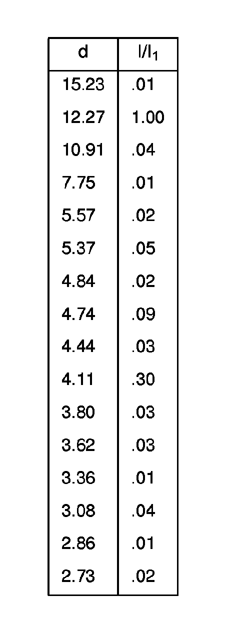

5. A process for preparing a crystalline mono(N,N'-dimethylformamide) solvate form of the compound of Formula I: 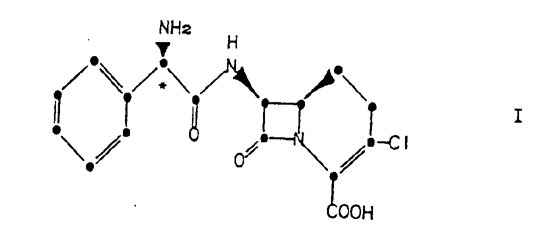 which comprises dissolving a compound of Formula I in aqueous N,N'-dimethylformamide, adjusting the pH to 5.9, adding acetonitrile, followed by filtration of the resulting solid and air drying at slightly above room temperature.

6. A process for preparing a compound of Claim 2, according to the process of claim 5.

## Patentansprüche

1. Kristalline Bis(N,N'-dimethylformamid)-Solvatform der Verbindung der Formel 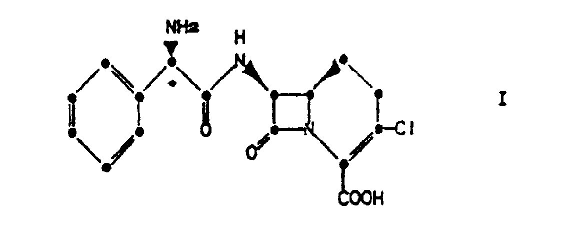 die das folgende Röntgenpulverbeugungsmuster hat, das mit einer durch Nickel gefilterten Kupferstrahlung mit λ _ = 1,5406 Å erhalten wurde, worin d die Netzebenenabstände und I/I₁ die relative Intensität bedeutet:

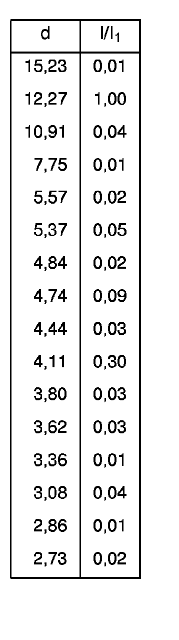

2. Kristalline Mono(N,N'-dimethylformamid)-Solvatform der Verbindung der Formel I: 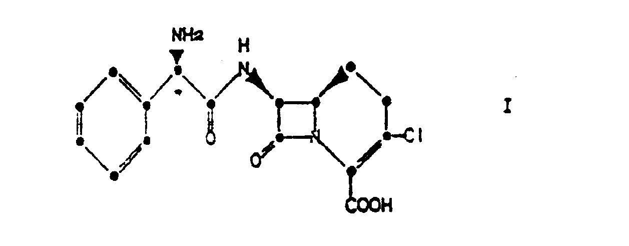 die das folgende Röntgenpulverbeugungsmuster hat, das mit einer durch Nickel gefilterten Kupferstrahlung mit λ = 1,5418 Å erhalten wurde, worin d die Netzebenenabstände und I/I₁ die relative Intensität bedeutet:

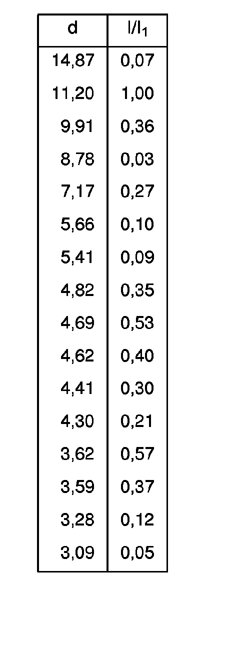

3. Verfahren zur Herstellung einer kristallinen Bis(N,N'-dimethylformamid)-Solvatform der Verbindung der Formel 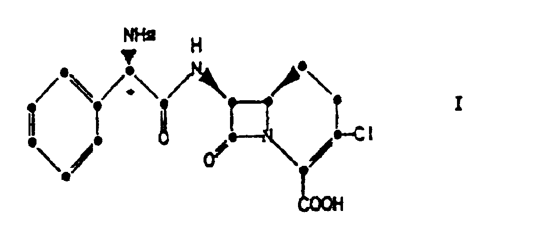 das umfaßt, daß man eine Verbindung der Formel I in wäßrigem N,N'-Dimethylformamid löst, den pH auf 5 bis 6 einstellt und anschließend filtriert und im Vakuum trocknet.

4. Verfahren nach Anspruch 3 zur Herstellung einer Verbindung nach Anspruch 1, die das folgende Röntgenpulverbeugungsmuster hat, das mit einer durch Nickel gefilterten Kupferstrahlung mit λ = 1,5406 Å erhalten wurde, worin d die Netzebenenabstände und I/I₁ die relative Intensität bedeutet:

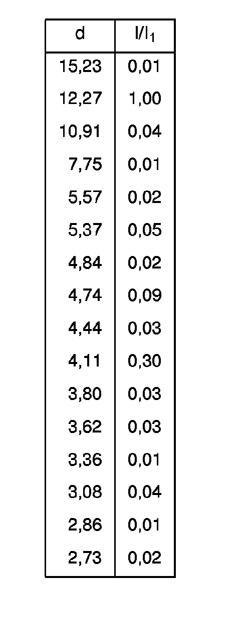

5. Verfahren zur Herstellung einer kristallinen Mono(N,N'-dimethylformamid)-Solvatform der Verbindung der Formel 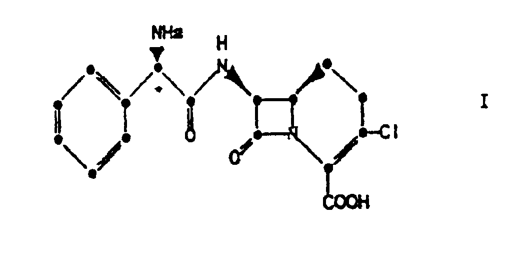 das umfaßt, daß man eine Verbindung der Formel in wäßrigem N,N'-Dimethylformamid löst, den pH auf 5,9 einstellt, Acetonitril zugibt, anschließend den entstehenden Feststoff filtriert und an der Luft etwas über Raumtemperatur trocknet.

6. Verfahren zur Herstellung einer Verbindung nach Anspruch 2 mit dem Verfahren von Anspruch 5.

## Revendications

1. Forme de solvate bis(N,N'-diméthylformamide) cristalline du composé répondant à la Formule 1 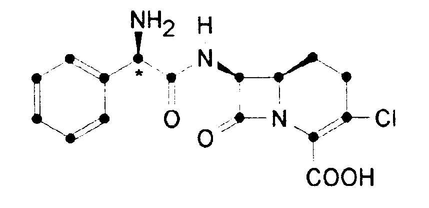 qui présente le diagramme de diffraction de la poudre par rayons X suivant, obtenu avec la radiation du cuivre filtré par le nickel λ = 1,5406 Å, dans lequel d représente l'écartement des plans et I/I₁ l'intensité relative :

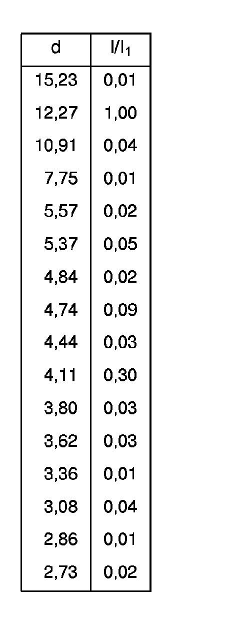

2. Forme de solvate mono(N,N'-diméthylformamide) cristalline du composé répondant à la Formule I 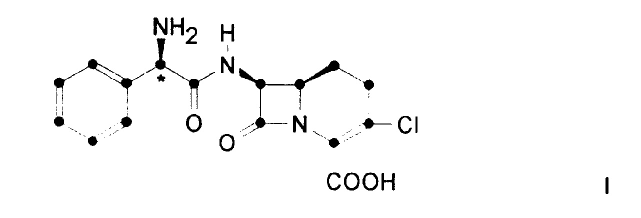 qui présente le diagramme de diffraction de la poudre par rayons X suivant, obtenu avec la radiation du cuivre filtré par le nickel λ = 1,5418 Å, dans lequel d représente l'écartement des plans et I/I₁ l'intensité relative :

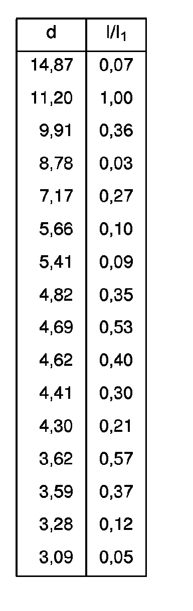

3. Procédé pour la préparation d'une forme de solvate bis(N,N'-diméthylformamide) cristalline du composé répondant à la Formule I 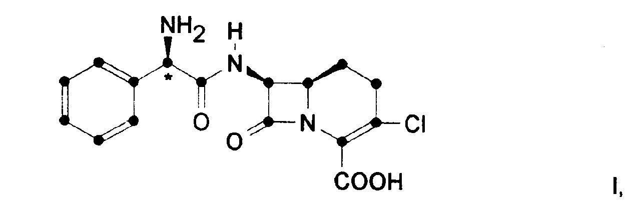 qui comprend la dissolution d'un composé répondant à la Formule I dans de la N,N'-diméthylformamide aqueuse, l'ajustement du pH à 5 à 6, suivi par la filtration et le séchage sous vide.

4. Procédé selon la revendication 3 pour la préparation d'un composé selon la revendication 1, qui présente le diagramme de diffraction de la poudre par rayons X suivant, obtenu avec la radiation du cuivre filtré par le nickel de λ = 1,5406 Å, dans lequel d représente l'écartement des plans et I/I₁ l'intensité relative :

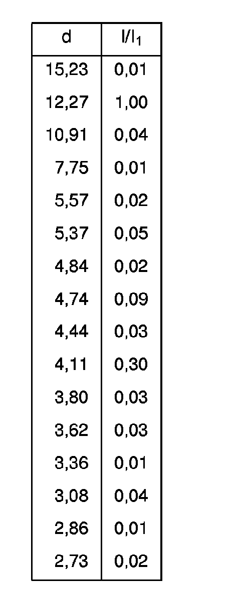

5. Procédé pour la préparation d'une forme de solvate mono(N,N'-diméthylformamide) cristalline du composé répondant à la Formule I : 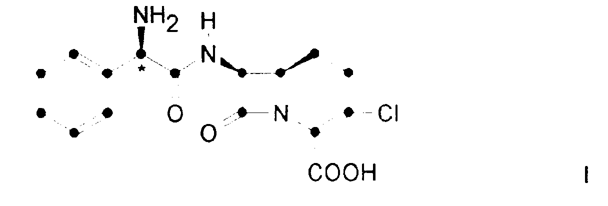 qui comprend la dissolution d'un composé répondant à la Formule I dans de la N,N'-dimétnylformamide aqueuse, l'ajustement du pH à 5,9, l'addition d'acétonitrile, suivie par la filtration du solide résultant et le séchage à l'air à une température légèrement supérieure à la température ambiante.

6. Procédé pour la préparation d'un composé selon la revendication 2 conformément au procédé selon la revendication 5.
